(19) Europäisches Patentamt
European Patent Office
Office européen des brevets

(11) Publication number: 0 007 648
B1

(12) # EUROPEAN PATENT SPECIFICATION

(45) Date of publication of patent specification: 11.08.82

(51) Int. Cl.³: C 07 D 295/12, C 07 D 413/12, C 07 D 213/82, A 61 K 31/535

(21) Application number: 79102782.4

(22) Date of filing: 02.08.79

(54) New acylureas with pharmaceutical activity, the process for their preparation, and pharmaceutical compositions which contain them.

(30) Priority: 02.08.78 ES 472295

(43) Date of publication of application:
06.02.80 Bulletin 80/3

(45) Publication of the grant of the patent:
11.08.82 Bulletin 82/32

(84) Designated Contracting States:
BE DE FR GB IT

(56) References cited:
FR - A - 2 011 593
FR - A - 2 115 209
FR - A - 2 143 361
FR - A - 2 348 207
GB - A - 969 022

(73) Proprietor: Investigacion Tecnica Y Aplicada, S.A.
Poligono Industrial Santiga calle Argenters, 6
Sta. Perpetua de Moguda Barcelona (ES)

(72) Inventor: Bruseghini, Leonida
calle Caponata, 5
Barcelona-34 (ES)
Inventor: Ribalta, José Miguel
125 bis calle Mayor de Sarriá
Barcelona (ES)
Inventor: Iniesta Pons, Jorge
39 calle Leyva
Barcelona (ES)

(74) Representative: Berg, Wilhelm, Dr.
Dr. Berg, Dipl.-Ing. Stapf, Dipl.-Ing. Schwabe, Dr.
Dr. Sandmair Mauerkircherstrasse 45
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 007 648 B1

New acylureas with pharmaceutical activity, the process for their preparation, and pharmaceutical compositions which contain them

This invention relates to new acylureas of the general formula I

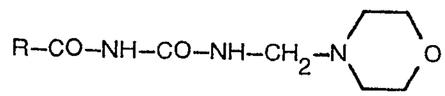

I

wherein R is one of the following groups:

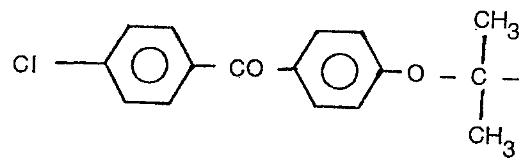

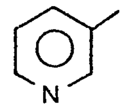

or

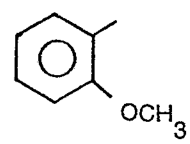

to give the compounds: N-2-[p-(p'-chlorobenzoyl)phenoxy]-2-methylpropionyl-N'-morpholinomethylurea (ITA 375), N-nicotinoyl-N'-morpholinomethylurea (ITA 378) and N-(2-methoxybenzoyl)-N'-morpholinomethylurea (ITA 418).

The procedure for obtaining the products of the general formula I is another object of this invention. This process comprises slowly adding an activated carboxylic acid derivative of the formula II

$$R—COX \qquad II$$

wherein R is the same as defined above, and X can be one of any of the halogens, or equally a group having the formula —O—CO—R (wherein R is the same as above), or equally a group having the formula —O—R' (wherein R' is a lower alkyl group) into a continuously stirred suspension of urea in a solvent which is organic, non-alcoholic and inert to the reagents, or, if the radical X has the formula —OR' into a suspension of monosodium urea or of urea in the presence of metallic sodium at a temperature of between ambient temperature and the boiling point of the solvent, keeping up the stirring and the heating for a period of between 1 and 24 hours, to isolate a precipitate which is an intermediate acylurea of the formula III

$$R—CO—NH—CO—NH_2 \qquad III$$

wherein R is the same as above, which precipitate, once washed with the suitable solvents is caused to react with morpholine and a suitable form of formaldehyde in a solvent whose polarity is not critical, to obtain the acylurea of the general Formula I by amino methylation.

The French Patent 2 348 207 discloses N-2-(p-chlorophenoxy)-isobutyryl-N'-morpholinomethylurea having hypocholesterolaemic, hypolipaemic and antiplatelet aggregation activity. The antiplatelet aggregation activity of the compounds of the invention reaches its maximum after 5 hours after oral administration whereas the maximum activity of the known compound is only obtained 17 hours after oral administration. This remarkable difference over the prior art is of great importance for obtaining a rapid pharmacological effect and could not be expected by the man skilled in the art.

The procedure for obtaining the acylurea of the general formula I is the following:

*Stage a.* — Obtention of the acylurea of the general formula III.

The activated carboxylic acid derivative II is added slowly to a continuously stirred suspension of urea in a non-alcoholic organic solvent inert to reagents, as for example benzene, toluene, xylene, pyridine or a chlorohydrocarbon derivative.

When the activated carboxylic acid derivative II is an acid halide, it is possible to use either an acid catalyst such as for example concentrated sulphuric acid, or alternatively, a base which acts as the acceptor of the hydrogen halide given off in the reaction such as for example pyridine or triethylamine, in which case it is appropriate to use at least the stoichiometric quantity with respect to the derivative II. The selection of the reaction temperature is not critical as the process can take place between ambient temperature and the solvent boiling point, the latter being preferable so as to accelerate the rate of reaction.

The stirring and the heating is kept up for a period varying from 1 to 24 hours after which the solution is allowed to cool at rest.

A crystalline precipitate is obtained which is washed with the same solvent as used in the reaction, followed with a solution of sodium bicarbonate then finally with water, thus obtaining the intermediate acylurea of the general formula III.

*Stage b.* — Obtention of the acylurea of general formula I

The acylurea thus obtained is made to react with morpholine and formaldehyde in a solvent the polarity of which is not critical, such that one can use water, acetic acid, dioxane, an alcohol such as ethanol or isoamyl alcohol or mixtures of these solvents in varying proportions; the medium can be slightly acid, neutral or basic and comprises preferably solvents with lower boiling points.

Formaldehyde may be used, either in the form of an aqueous solution, preferably in a concentration between 35 and 40% (formalin) or in the form of trioxymethylene (in which case the medium must be slightly acid and the reaction times extended) or in the form of paraformaldehyde (polyoxymethylene) in any of its forms.

The mixture of the acylurea III, morpholine, formaldehyde and the solvent is taken to the boiling temperature of the solvent and is so kept during a period varying between 1 and 24 hours.

The product is isolated either by crystallization and filtration of the cold crude reaction product or by precipitation followed by filtration of the crude product poured onto an excess of water.

The product so obtained in a yield of approximating to 90% is purified by obtaining the corresponding hydrochloride. For this one dissolves the product in an organic solvent such as alcohol, dioxane, or a chlorohydrocarbon and causes to bubble a stream of hydrogen chloride through the organic solution until total precipitation, or one adds a stoichiometric quantity of concentrated hydrochloric acid to said solution.

The precipitate obtained is filtered and washed with the same solvent as was used to prepare the solution.

To obtain again the product I in a free base form starting from a hydrochloride a stoichiometric quantity of sodium hydroxide or potassium hydroxide in aqueous solution is added to a continuously stirred suspension of the hydrochloride in an organic solvent which does not mix with water, such as methylene chloride or benzene. The aqueous phase is then decanted and the organic phase washed with water, dried with sodium sulphate or another appropriate drying agent, whereafter the solvent is removed and a high purity product is thus obtained.

Below are described several examples of the obtention of the new acylureas of the general formula I.

## Example 1

Obtention of the N-2-[p-(p′-chlorobenzoyl)-phenoxy]-2-methylpropionyl-N′-morpholinomethylurea (also referred to as ITA 375)

*Stage a.* — Obtention of N-2-[p-(p′-chlorobenzoyl)-phenoxy]-2-methylpropionylurea

A suspension of 37,1 g (0,618 m) of urea in 250 ml of anhydrous benzene is caused to reflux. Drop by drop a solution of 48,3 g (0,143 m) of 2-[p-(p′-chlorobenzoyl)-phenoxy]-2-methylpropionyl-chloride in 80 ml of anhydrous benzene is added and the refluxing of the mixture is continued for 12 hours.

On cooling the product is crystallized and is filtered, washed with benzene, with a saturated solution of sodium bicarbonate and finally with water, and then vacuum dried with calcium chloride.

This produces 31,0 g (60%) of a white solid of f.p. = 173—185°C. Recrystallizing in ethanol produces a product of f.p. = 183—186°C.

TLC ($Cl_2CH_2$/AcOEt; 1/1); Single spot

IR (KBr): Strong peaks at 3395, 3355 and 3215 (N—H), 1708 and 1693 (C=O), 1597, 1380 and 1147 $cm^{-1}$.

*Stage b.* — Obtention of N-2-p-(p′-chlorobenzoyl)-phenoxy-2-methylpropionyl-N′-morpholinomethylurea (ITA 375)

The reaction is the following:

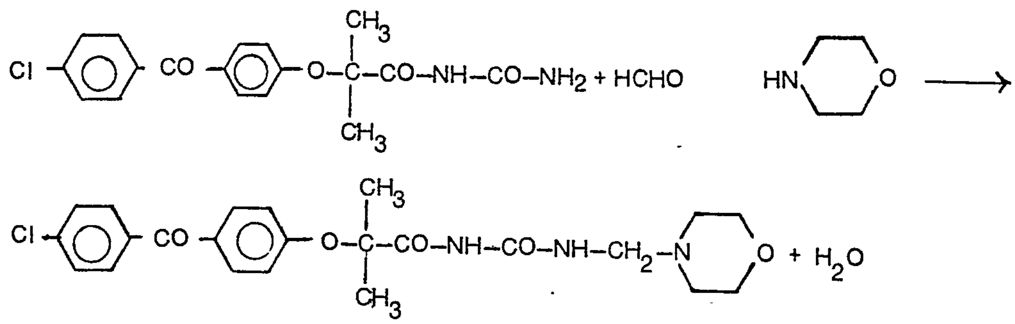

A mixture of 21,0 g (0,058 m) of N-2-[p-(p′-chlorobenzoyl)-phenoxy]-2-methylpropionylurea, 10,1 g (0,116 m) of morpholine and 19,7 g (0,233 m) of 35% aqueous formaldehyde in 84 ml of dioxane and 126 ml of water is made to reflux for 10 hours. It is left to cool to ambient temperature and is poured on to 1000 ml of water. It is left in the refrigerator and there is precipitated a solid paste which once separated by decantation is dissolved in methylene-chloride, washed with water and dried with sodium sulphate. Evaporating the solvent leaves an oil which is purified via the hydrochloride. For this the oil is dissolved in dioxane and HCl gas is passed through the solution precipitating the hydrochloride which is separated by filtration.

The reconversion to the free base is achieved by suspending the hydrochloride precipitate in methylene chloride and treating it with a stoichiometric quantity of NaOH(2N).

Evaporating the organic phase until dry leads to 10,8 g (40%) of a crystalline white solid of f.p. = 130,5—131,5°C.

TLC: ($Cl_2CH_2$/AcOEt; 1/1) single spot.
Potentiometric evaluation of basic groups: 99,8%.
IR (KBr): Strong peaks at 3350, 3218 and 3140 (N—H), 1696 (C=O), 1240, 1150 and 768 $cm^{-1}$.
NMR ($CDCl_3$): $\delta$ 8,69 (m, 1, CO*NH*CO); $\delta$ 8,55 (m, 1, *NH*$CH_2$); $\delta$ 7,3 (m, 8, aromatics); $\delta$ 4,40 (d, 2, NH*$CH_2$*); $\delta$ 3,63 (t, 4, *$CH_2$*—O—*$CH_2$*); $\delta$ 2,51 (t, 4, *$CH_2$*—N—*$CH_2$*); $\delta$ 1,53 (s, 6, $CH_3$).

## Example 2

Obtention of N-nicotinoyl-N′-morpholinomethylurea (also referred as ITA—378)

*Stage a.* — Obtention of N-nicotinoylurea

To a solution of 62,5 g (1,041 m) of urea in 750 cc of dry pyridine 125,0 g (0,702 m) nicotinoyl chloride-hydrochloride is added. Thereafter it is gently heated to reflux and maintained during 7,5 hours.

After cooling the precipitate formed is separated by filtration, washed with pyridine, with the least quantity of water and with boiling ethanol, and then vacuum dried over sulphuric acid. Thereby 24,6 g (21%) of a white solid are obtained of f.p. = 221—224°C.

TLC, (Acetone): single spot.
Potentiometric evaluation of acid groups: 98,4%.
IR (KBr): Strong peaks at 3402, 3154 (N—H), 1714 and 1678 (C=O), 1392, 1283 and 1106 $cm^{-1}$.

*Stage b.* — Obtention of N-nicotinoyl-N′-morpholinomethylurea (ITA—378)

The reaction is the following:

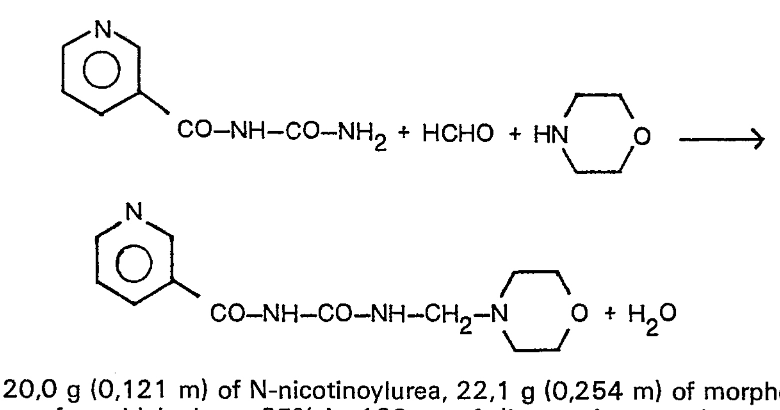

A mixture of 20,0 g (0,121 m) of N-nicotinoylurea, 22,1 g (0,254 m) of morpholine and 23,9 g (0,279 g) of aqueous formaldehyde at 35% in 100 cc of dioxane is caused to reflux for 8 hours. Thereafter 300 cc of ethyl ether is added and the mixture is left to crystallize in the refrigerator. A solid paste is obtained which is crystallized in a mixture of methanol and ethyl ether. Finally 7,4 g (23%) of a white crystalline solid of f.p. = 148—153°C is obtained.

TLC (Acetone): Single spot with traces of the unreacted starting product.
Potentiometric evaluation of the basic groups: 95,3%.
IR (KBr): Strong peaks at 3300, 3247, 3152 (N—H), 1692, 1670 (C=O), 1540, 1280, 1118 and 1010 $cm^{-1}$.
NMR ($CDCl_3$): $\delta$ 9,25 (s, 1, NC*H*CCO); $\delta$ 9,18 (m, 1, *NH*$CH_2$); $\delta$ 8,80 (d, 1, NC*H*CH); $\delta$ 9,34 (d, 1, CHC*H*CCO); $\delta$ 7,41 (t, 1, CHC*H*CH); $\delta$ 4,24 (d, 2, NH$CH_2$); $\delta$ 3,68 (t, 4, $CH_2$—O—$CH_2$); $\delta$ 2,62 (t, 4, $CH_2$—N—$CH_2$).

Example 3

Obtention of N-(2-methoxybenzoyl)-N'-morpholinomethylurea (ITA—418)

*Stage a.* — Obtention of N-(2-methoxybenzoyl)-urea.

63,7 g (1,061 m) of urea in 380 cc of dry pyridine are caused to reflux whereafter 127,4 (0,747 m) of 2-methoxybenzoylchloride are slowly added. The refluxing is maintained for 3 hours.

The material is cooled and precipitated in 1000 cc of 2 N HCl and ground ice. The formed precipitate is separated by filtration, washed with water and vacuum dried on $CaCl_2$ at 50°C.

77,0 g (53%) of a white solid of f.p. = 179—184°C are obtained.

TLC (AcOEt): single spot.
IR (KBr): Strong peaks at 3356 and 3210 (N—H), 1719 and 1670 (C=O), 1482, 1370 and 1292 $cm^{-1}$.

*Stage b.* — Obtention of N-(2-methoxybenzoyl)-N'-morpholinomethylurea (ITA—418).

The reaction is the following:

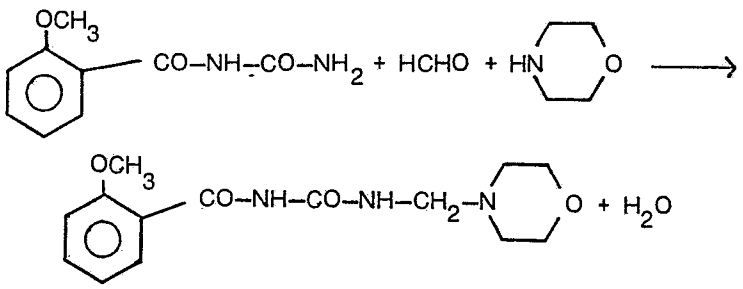

A mixture of 46,5 g (0,240 m) of N-(2-methoxybenzoyl)-urea 52,1 g (0,600 m) of morpholine and 51,3 (0,600 m) of 35% aqueous formaldehyde in 400 cc of dioxane is refluxed for 8 hours. Part of the solvent is evaporated so as to reduce the volume to half. Then the solution is poured on to hot hexane and stirred. An oil is formed which separates on decanting the hexane. The oil is dissolved in ethanol and the solution is treated with anhydrous sodium sulphate and active carbon and then filtered.

The liquid obtained is cooled in a bath of ice and gaseous HCl is passed through the liquid for a duration of 8 hours. A white hydrochloride precipitate is collected which when washed and dried weighs 41,3 g (52%) and has a f.p. = 160—164°C (d).

To reconvert to the free base the hydrochloride is suspended in methylene chloride, treated with 1N NaOH in stoichiometric quantity. Evaporating the solvent from the organic phase leads to a colourless oil which crystallizes gradually to a crystalline solid with a f.p. = 91—93,5°C.

TLC (AcOEt/Acetone; 4/1): single spot.
Potentiometric evaluation of basic groups: 99,5%.
IR (KBr): strong peaks at 3347, 3304 (N—H); 1700, 1663 (C=O), 1475, 1300, 1115 and 1005 $cm^{-1}$.
NMR ($CDCl_3$): $\delta$ 9,86 (m, 1, CO*NH*CO); $\delta$ 9,04 (m, 1, *NH*$CH_2$); $\delta$ 8,16 (d, 1 arom. 6); $\delta$ 7,76—7,30 (m, 1, arom 4); $\delta$ 7,30—6,80 (m, 2, arom 3 and 5); $\delta$ 4,24 (d, 2, NH$CH_2$); $\delta$ 3,97 (s, 3, $OCH_3$); $\delta$ 3,68 (t, 4, $CH_2$—O—$CH_2$); $\delta$ 2,62 (t, 4, $CH_2$—N—$CH_2$).

PHARMACOLOGICAL ACTIVITY

The compounds of the general formula I obtained in accordance with the procedure of the present invention possess to a greater or lesser degree the property of inhibiting the platelet aggregation in humans and animals as well as hypocholesterolaemic and hypolipaemic activity. Below are summarized the first proofs of the pharmacological activity of the products I.

*Toxicity*

The respective DL50 have been evaluated in NMRI male mice by the method of J. T. Litchfield and F. Wilcoxon (Journal of Pharmacology and Experimental Therapeutics *98* (1949) 99).

The products were administered intraperitoneally suspended in Tween 80 (Trade Mark) at 1% plus CMC (carboxymethylcellulose) at 2% in physiological serum.

The values obtained are shown in Table I below.

The reference standard use was phenylbutazone.

*Anti platelet aggregation activity*

The main activity of the products I is that of inhibiting platelet aggregation.

The test was conducted "ex vivo" in accordance with the procedures of Born (Nature *194* (1962) 927) by administering the products by gastric catheter to rats (Sprage Dawley) of both sexes of a weight between 300 and 500 g.

Platelet aggregations are induced by ADP (adenosindiphosphate) and measured in an aggregometer (Bryston) connected to a model R Omniscriber recorder. The antiaggregation activity measured after 5 hours from the administration the product as a % of inhibition is compared in Table I below with the activity of a double dose of acetylsalicylic acid.

Products ITA 375 and 378 stand out in their anti platelet aggregation activity, similar to that of the acetylsalicylic acid given in a double dose.

TABLE I

Toxicity and anti platelet – aggregation activity

| Product | Reference | LD50 mg/kg i.p. NMRI mouse | Anti platelet aggregation p.o. Dose mg/kg | % inhibition (5 hours) |
|---|---|---|---|---|
| N-2-[p-(p'-chlorobenzoyl)phenoxy]-2-methylpropionyl-N'-morpholinomethylurea | ITA–375 | 950 | 200 | 68,5 |
| N-nicotinoyl-N'-morpholinomethylure | ITA–378 | 1700 | 200 | 81,9 |
| N-(methoxybenzoyl)-N-morpholinomethyl-urea | ITA–418 | 1100 | 200 | 68,0 |
| Phenylbutazone | – | 154 | – | – |
| Acetylsalicylic acid | – | – | 400 | 86,3 |

*Hypocholesterolaemic activity and hypolipaemic activity*

To determine the hypocholesterolaemic activity tests have been conducted with hypercholesterolaemia induced by the administration of Triton WR 1339 (Trade Mark) in accordance with S. Garattini, C. Morpurgo and Passerini (Experiment *12* (1956) 347). The results obtained using clofibrate (ethyl ester of p-chlorophenoxyisobutyric acid), as a reference standard are shown in Table II below.

For this hypocholesterolaemic activity, among the products examined, that with the reference ITA—375 stands out for its efficacity. For the determination of the hypolipaemic activity the products were administered orally suspended in 10 ml/kg of olive oil, evaluating the lactescence of the serum 6 hours after administration. The results obtained against clofibrate are alsc shown in Table II below.

As may be observed the products under reference ITA—375 and ITA—378 stand out in this activity.

TABLE II

Hypocholesterolaemic and hypolipaemic activities

| Reference | Dose (mg/kg) | Activity: hypo-cholesterolaemic (%) | Activity: hypolipaemic (%) |
|---|---|---|---|
| ITA–375 | 200 | 29,1 | 41,1 |
| ITA–378 | 200 | 12,9 | 54,7 |
| ITA–418 | 200 | 10,4 | – |
| Clofibrate | 200 | 13,8 | 22,8 |

DOSIFICATION

The dose of the active pharmaceutical product can vary between 500 and 1000 mg per unit of administration.

FORMS OF ADMINISTRATION

The above described products of the general formula I of the present invention can be administered in the form of injections, capsules, coated or uncoated pills, syrups, drops or other suitable forms.

The pharmaceutical speciality which contains the active compound can be prepared conveniently using one or other of the various organic or inorganic vehicles.

Among the pharmaceutical vehicles are included for example, water, mixtures of water and hydrosoluble solvents such as alkanols, aralkanols of low molecular weight, oils and vegetables, polyalkyleneglycols, acetylcellulose, carboxymethylcellulose, polyvinylpyrrolidone, Avicel (Trade Mark), Aerosil (Trade Mark), magnesium stearate, talc and/or Pharmacoat (Trade Mark).

The pharmaceutical speciality may also contain ancilliary non-toxic substances such as dispersing, compacting, emulsifying, preservative, wetting agents or others with particularly desirable action, such as, for example, polyethyleneglycol 200, 300, 400 and 600, Carbowaxes 1000, 1500, 4000 and 10,000 (Trade Marks), tamponing ingredients such as sodium chloride, sodium borate, sodium acetate, gluconate, and other ingredients of a conventional type as sorbitan monolaurate, triethanolamine oleate, polyoxyethylenesorbitan monopalmitate, sodium dioctylsulfosuccinate, monothioglycerol, thiosorbitol, ethylenediaminotetraacetic acid and others similar.

Respective examples of suitable pharmaceutical compositions containing the active components of the general formula I are the following:

Example 4 — Capsules

Capsules with 500 mg of active component

| | |
|---|---|
| 1 capsule | |
| active component | 500 mg |
| Aerosil 200 (Trade Mark) | 10 mg |
| magnesium stearate | 10 mg |
| Avicel (Trade Mark) | 100 mg |

Example 5 — Pills

Pills with 500 mg of active component

| | |
|---|---|
| Active component | 500 mg |
| Aerosil 200 (Trade Mark) | 10 mg |
| Avicel 101 (Trade Mark) | 230 mg |
| magnesium stearate | 10 mg |
| talc | 50 mg |

10 mm diameter pills are obtained by double compacting.

*Therapeutical use*

The compounds of the general formula I may be used for the treatment of cases of vasculopaties, infarct or danger of infarct, thrombosis of the artery or vein, embolism, vascular and lymphatic prosthesis implantation, extracorporeal circulation disorders of the artery and venous circulation, hypercoagulation, platelet hyperaggregation, and dangers of artery and venous thrombosis.

**Claims**

1. Acylureas with pharmacological activity of the general formula I:

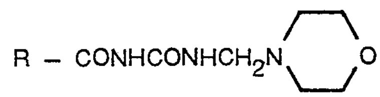 I

wherein R is one of the following groups:

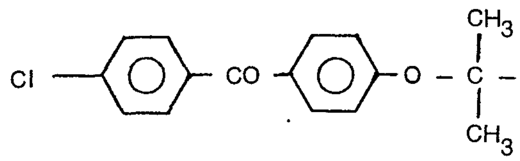

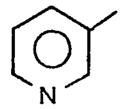

or

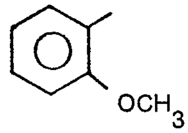

2. A process for preparing the acylureas as defined in Claim 1, characterized by slowly adding an activated carboxylic acid derivative of the formula II

R—COX II

wherein R is the same as defined in Claim 1, and X can be one of any of the halogens, or equally a group having the formula —O—CO—R (wherein R is the same as above), or equally a group having the formula —O—R′ (wherein R′ is a lower alkyl group) into a continuously stirred suspension of urea in a solvent which is organic, non-alcoholic and inert to the reagents, or, if the radical X has the formula —OR′ into a suspension of monosodium urea or of urea in the presence of metallic sodium at a temperature of between ambient temperature and the boiling point of the solvent, keeping up the stirring and the heating for a period of between 1 and 24 hours, to isolate a precipitate which is an intermediate acylurea of the formula III:

$R—CO—NH—CO—NH_2$ III

wherein R is the same as above, which precipitate, once washed with the suitable solvents is caused to react with morpholine and a suitable form of formaldehyde in a solvent whose polarity is not critical, to obtain the acylurea of the general formula I by amino methylation.

3. The process as defined in Claim 2, characterized by using an acid catalyst in conjunction with an acid halide of the general formula II in the reaction to obtain the acylurea of the general formula III.

4. The process as defined in Claim 2, wherein the compound of general formula II is an acid halide, characterized by using a basic catalyst in a stoichiometric quantity with respect to the acid halide, so that it acts as acceptor of the hydrogen halide released in the reaction producing the compound of the general formula III.

5. The process as defined in the Claims 2, 3 or 4, characterized by carrying out the reaction for obtaining the acylurea of the general formula III at the boiling temperature of the solvent.

6. The process as defined in the Claims 2, 3, 4 or 5, characterized by using water, acetic acid, dioxane, an alcohol or mixture of these solvents in varying proportions as solvent in the stage of obtaining the acylurea of the general formula I starting from the acylurea of the general formula III.

7. The process as defined in the Claims 2, 3, 4, 5 or 6, characterized by carrying out the reaction for the obtention of the acylurea of the general formula I starting from the acylurea of the general formula III at the boiling temperature of the solvent.

8. The process as defined in Claims 2, 3, 4, 5, 6 or 7, characterized by using the formaldehyde in the form of formalin or in the form of trioxymethylene, in which case the medium must be slightly acid and the reaction time extended, or in the form of paraformaldehyde (polyoxymethylene) in any of its forms.

9. The process as defined in the Claims 2, 3, 4, 5, 6, 7 or 8, characterized by purifying the acylurea of the general formula I by formation of the corresponding hydrochloride and its subsequent reconversion to the free base.

10. Pharmaceutical composition characterized by having as an active component one of the acylureas with pharmacological activity of the general formula I as defined in Claim 1.

## Revendications

1. Acylurées à activité pharmacologique de formule générale I:

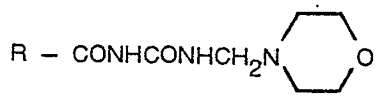 I

dans laquelle R représente l'un des groupements suivants:

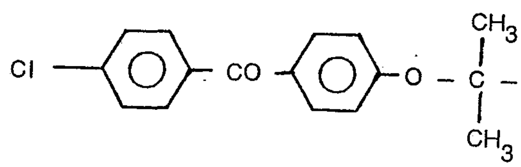

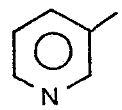

ou

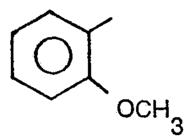

2. Procédé de préparation des acylurées telles que définies dans la revendication 1, caractérisé par le fait que l'on ajoute lentement un dérivé d'acide carboxylique activé de formule II:

$$R—COX \qquad II$$

dans laquelle R est défini comme dans la revendication 1, et X peut être l'un quelconque des halogènes, ou également un groupement de formule —O—CO—R (R étant tel que défini ci-dessus) ou également un groupement de formule —O—R' (R' étant un groupement alkyle inférieur) à une suspension continuellement agitée d'urée dans un solvant organique, non alcoolique, et inerte vis-à-vis des réactifs, ou, si le radical X représente la formule —OR', dans une suspension de monosodium urée ou d'urée en présence de sodium métallique à une température entre la température ambiante et la température d'ébullition du solvant, que l'on continue l'agitation et le chauffage pendant une période comprise entre 1 heure et 24 heures, pour isoler un précipité qui est une acylurée intermédiaire de formule III:

$$R—CO—NH—CO—NH_2 \qquad III$$

dans laquelle R est défini comme ci-dessus, et que ledit précipité, une fois lavé avec les solvants convenables, est mis en réaction avec la morpholine et une forme convenable de formaldéhyde dans un solvant dont la polarité n'est pas critique, pour obtenir l'acylurée de formule générale I par aminométhylation.

3. Procédé selon la revendication 2, caractérisé par le fait que l'on utilise un catalyseur acide en combinaison avec un halogénure d'acide de formule générale II dans la réaction d'obtention de l'acylurée de formule générale III.

4. Procédé selon la revendication 2, dans lequel le composé de formule générale II est un halogénure d'acide, caractérisé par le fait que l'on utilise un catalyseur basique en quantité stoechiométrique par rapport à l'halogénure d'acide, de façon qu'il agisse comme accepteur de l'acide halohydrique formé dans la réaction produisant le composé de formule générale III.

5. Procédé tel que défini dans les revendications 2, 3 ou 4, caractérisé par le fait que l'on effectue la réaction d'obtention de l'acylurée de formule générale III à la température d'ébullition du solvant.

6. Procédé tel que défini dans les revendications 2, 3, 4 ou 5, caractérisé par le fait que l'on utilise de l'eau, de l'acide acétique, du dioxane, un alcool ou un mélange de ces solvants en proportions variables, comme solvant dans l'étape d'obtention de l'acylurée de formule générale I au départ de l'acylurée de formule générale III.

7. Procédé tel que défini dans les revendications 2, 3, 4, 5 ou 6, caractérisé par le fait que l'on effectue la réaction pour l'obtention de l'acylurée de formule générale I au départ de l'acylurée de formule générale III à la température d'ébullition du solvant.

8. Procédé tel que défini dans les revendications 2, 3, 4, 5, 6 ou 7, caractérisé par le fait que l'on utilise le formaldéhyde sous la forme de formaline ou sous la forme de trioxyméthylène, auquel cas le milieu doit être légèrement acide et le temps de réaction augmenté, ou sous la forme de paraformaldéhyde (polyoxyméthylène) dans l'une quelconque de ses formes.

9. Procédé tel que défini dans les revendications 2, 3, 4, 5, 6, 7 ou 8, caractérisé par le fait que l'on purifie l'acylurée de formule générale I par formation du chlorhydrate correspondant que l'on reconvertit ensuite en base libre.

10. Composition pharmaceutique, caractérisée par le fait qu'elle possède comme ingrédient actif une des acylurées à activité pharmacologique de formule I telles que définies dans la revendication 1.

**Patentansprüche**

1. Acylharnstoffe mit pharmakologischer Aktivität der allgemeinen Formel I

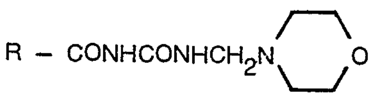

I

in der
R eine der folgenden Gruppen:

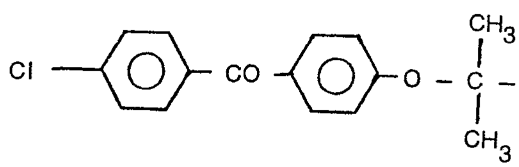

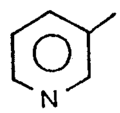

oder

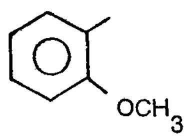

bedeutet.

2. Verfahren zur Herstellung der in Anspruch 1 definierten Acylharnstoffe, dadurch gekennzeichnet, daß man ein aktiviertes Carbonsäure-derivat der Formel II

$$R—COX \qquad II$$

worin R die in Anspruch 1 angegebenen Bedeutungen besitzt und X eines der Halogene oder auch eine Gruppe der Formel —O—CO—R (worin R die oben angegebenen Bedeutungen besitzt) oder auch eine Gruppe der Formel —O—R' (worin R' eine niedrige Alkylgruppe darstellt) bedeutet, langsam zu einer kontinuierliche gerührten Suspension von Harnstoff in einem organischen, nichtalkoholischen und gegenüber den Reaktionsteilnehmern inerten Lösungsmittel oder, wenn die Gruppe X der Formel —OR' entspricht, zu einer Suspension von Mononatriumharnstoff oder Harnstoff in Gegenwart von metallischem Natrium bei einer Temperatur zwischen Raumtemperatur und dem Siedepunkt des Lösungsmittels zusetzt, das Rühren und Erhitzen während einer Zeitdauer zwischen 1 und 24 Stunden aufrechterhält, um einen Niederschlag zu isolieren, der ein Zwischenprodukt-Acylharnstoff der Formel III

$$R—CO—NH—CO—NH_2 \qquad III$$

darstellt, worin R die oben angegebenen Bedeutungen besitzt, welchen Niederschlag, man nach dem Waschen mit geeigneten Lösungsmitteln mit Morpholin und einer geeigneten Form von Formaldehyd in einem Lösungsmittel, dessen Polarität nicht kritisch ist, umsetzt, um den Acylharnstoff der allgemeinen Formel I durch Aminomethylierung zu bilden.

3. Verfahren nach Anspruch 2, dadurch gekennzeichnet, daß man bei der Reaktion zur Bildung des Acylharnstoffs der allgemeinen Formel III einen sauren Katalysator zusammen mit einem Säurehalogenid der allgemeinen Formel II verwendet.

4. Verfahren nach Anspruch 2, wobei die Verbindung der allgemeinen Formel II ein Säurehalogenid darstellt, dadurch gekennzeichnet, daß man einen basischen Katalysator in stöchiometrischer Menge, bezogen auf das Säurehalogenid verwendet, so daß er als Akzeptor für den Halogenwasserstoff wirkt, der während der Reaktion unter Bildung der Verbindung der allgemeinen Formel III freigesetzt wird.

5. Verfahren nach den Ansprüchen 2, 3 oder 4, dadurch gekennzeichnet, daß man die Reaktion zur Bildung des Acylharnstoffs der allgemeinen Formel III bei der Siedetemperatur des Lösungsmittels durchführt.

6. Verfahren nach den Ansprüchen 2, 3, 4 oder 5, dadurch gekennzeichnet, daß man Wasser, Essigsäure, Dioxan, einen Alkohol oder eine Mischung dieser Lösungsmittel in variierende Verhältnissen als Lösungsmittel in der Stufe der Bildung des Acylharnstoffs der allgemeinen Formel I ausgehend von dem Acylharnstoff der allgemeinen Formel III verwendet.

7. Verfahren nach den Ansprüchen 2, 3, 4, 5 oder 6, dadurch gekennzeichnet, daß man die Reaktion zur Bildung des Acylharnstoffs der allgemeinen Formel I ausgehend von dem Acylharnstoff der allgemeinen Formel III bei der Siedetemperatur des Lösungsmittels durchführt.

8. Verfahren nach den Ansprüchen 2, 3, 4, 5, 6 oder 7, dadurch gekennzeichnet, daß man den Formaldehyd in Form von Formalin oder in Form von Trioxymethylen, in welchem Fall das Medium schwach sauer sein und die Reaktionszeit verlängert werden muß, oder in Form von Paraformaldehyd (Polyoxymethylen) in irgendeiner seiner Formen verwendet.

9. Verfahren nach den Ansprüchen 2, 3, 4, 5, 6, 7 oder 8, dadurch gekennzeichnet, daß man den Acylharnstoff der allgemeinen Formel I durch Bildung des entsprechenden Hydrochlorids und dessen anschließende Umwandlung in die freie Base reinigt.

10. Pharmazeutische Zubereitung, dadurch gekennzeichnet, daß sie als Wirkstoff einen der Acylharnstoffe mit pharmakologischer Aktivität der allgemeinen Formel I, wie sie in Anspruch 1 definiert sind, enthält.